# EUROPEAN PATENT SPECIFICATION

(11) **EP 3 886 815 B1**
(45) Date of publication and mention of the grant of the patent: **02.11.2022**
(21) Application number: 19839631.9
(22) Date of filing: 28.11.2019
(51) Int. Cl.: A61K 9/20, A23L 5/00, A23L 33/16, A23L 33/135

(54) **MINERAL ELEMENTS-COMPRISING HAFNIA ALVEI COMPOSITIONS**
HAFNIA ALVEI-ZUSAMMENSETZUNGEN, DIE MINERALISCHE ELEMENTE ENTHALTEN
COMPOSITIONS D'HAFNIA ALVEI COMPRENANT D'ÉLÉMENTS MINÉRAUX

(30) Priority: 28.11.2018 EP 18208985
(43) Date of publication of application: 06.10.2021
(73) Proprietor: Targedys, 91160 Longjumeau (FR)
(72) Inventor: PICOLO, Clémentine, 76000 Rouen (FR); LAMBERT, Grégory, 92290 Châtenay-Malabry (FR)
(74) Representative: Icosa
(86) International application number: PCT/EP2019/082951
(87) International publication number: WO 2020/109492

(56) References cited:
- EP-A1- 1 228 769
- WO-A1-2017/174658
- CN-A- 102 125 179
- US-A1- 2007 184 153

## Description

### FIELD OF INVENTION

The present invention relates to probiotic strain compositions, namely *Hafnia alvei* compositions comprising zinc bisglycinate and/or chrome picolinate, and oral formulations thereof

### BACKGROUND OF INVENTION

The international WO2017/174658 patent application discloses pharmaceutical and food compositions comprising *Hafnia alvei* for inducing satiation prolonging satiety and improving body-weight composition in subjects in need thereof.

Pharmaceutical and nutraceutical compositions aiming the prevention or the treatment of metabolism-related disorders often comprise zinc and chrome. Nevertheless, such mineral elements may be deleterious for the probiotic strains, such as *Hafnia alvei,* comprised in such compositions.

Indeed, preliminary data of the Applicant have shown that *Hafnia alvei,* as many probiotic strains, are vulnerable towards ionic strength modifications in the vehicle they are carried. Therefore, suitable compositions and formulations comprising *Hafnia alvei* in association with zinc and chrome salts is an unmet need.

It was surprisingly found by the Applicant, the association of *Hafnia alvei* with particular organic salts of zinc and chrome according to the present invention, have no effect in the viability of the probiotic strain.

### SUMMARY

This invention thus relates to a pharmaceutical or nutraceutical composition, comprising a *Hafnia alvei* strain probiotic composition; said strain expressing the ClpB protein; in association with zinc bisglycinate and/or chrome picolinate.

In one embodiment, chrome picolinate is in an amount ranging from 0.01 to 0.04 % (w/w), in weight relative to the composition.

In one embodiment, zinc bisglycinate is in an amount ranging from 2 to 4 % (w/w), in weight relative to the composition.

In a further embodiment of the invention, the ClpB protein in the *Hafnia alvei* probiotic composition is in an amount of at least 0.7 % (w/w) in weight relative to the total weight of said composition; and the ratio of the total number of *Hafnia alvei* Colony Forming Units to the total *Hafnia alvei* cell number is at least 10⁻⁴.

In one embodiment, the number of *Hafnia alvei* Colony Forming Units cells is equal or superior to 10⁶ per gram of the probiotic composition.

In one embodiment, the total number *Hafnia alvei* cell number is equal or superior to 10¹⁰ per gram of the probiotic composition.

In one embodiment, the *Hafnia alvei* probiotic composition is freeze-dried.

In one embodiment, the pharmaceutical or nutraceutical composition further comprises at least one pharmaceutically or nutraceutically acceptable excipient; preferably said excipient being selected from at least one anti-adherent and at least one texturizing agent. Preferably, the anti-adherent is magnesium stearate and the texturizing agent is a modified starch.

In one particular embodiment, the composition comprises:
- from 10% to 15 %(w/w) of a *Hafnia alvei* composition as previously described;
- from 80 to 85 % (w/w) of modified starch;
- from 0.5 to 1.5 % (w/w) of magnesium stearate;
- from 2.0 to 3.0 % (w/w) of a zinc organic salt selected from zinc bisglycinate; and
- from 0.01 to 0.03 % (w/w) of a chrome organic salt selected from chrome picolinate;

The invention further relates to oral dosage forms comprising the pharmaceutical or nutraceutical composition of the invention. Preferably the dosage form is selected from capsules and tablets, even more preferably the dosage form is a capsule.

In one preferred embodiment, the oral dosage form of the invention is coated with an enteric coating. Preferably said enteric coating comprises hydroxypropyl methyl-cellulose and gellan gum.

In a last aspect, the invention relates to a blister comprising at least one oral dosage form according to the invention.

### DEFINITIONS

In the present invention, the following terms have the following meanings:
- **"About"** preceding a figure means plus or less 10% of the value of said figure.
- **"chromium picolinate"** designates chromium(3+);pyridine-2-carboxylate C₁₈H₁₂CrN₃O₆.CAS number 14281-83-5.
- **"Food composition", "dietary supplements", "nutraceutical composition"** and **"functional food"** are interchangeable and refer to any substance containing nutrients, whether for human or animal consumption, whether comprised of a single ingredient or a mixture of ingredients, whether liquid, liquid containing or solid, whether primarily carbohydrate, fat, protein or any mixture thereof, whether edible per se or requiring processing like cooking, mixing, cooling, mechanical treatment and the like.
- **"Pharmaceutically"** or **"nutraceutically acceptable"** refer to molecular entities and compositions that do not produce an adverse, allergic or other untoward reaction when administered to a subject, especially a human, as appropriate. A pharmaceutically acceptable carrier or excipient refers to a non-toxic solid, semi-solid or liquid filler, diluent, encapsulating material or formulation auxiliary of any type. Pharmaceutically or nutraceutically acceptable excipients that may be used in the compositions of the invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, silica, colloidal silica, magnesium trisilicate, polyvinyl pyrrolidone, cellulose-based substances (for example sodium carboxymethylcellulose), modified starches, polyethylene glycol, polyacrylates, waxes, polyethylene- polyoxypropylene- block polymers, polyethylene glycol and wool fat. In the pharmaceutical or nutraceutical compositions of the present invention, the active principle, alone or in combination with another active principle, can be administered in a unit administration form, as a mixture with conventional pharmaceutical or nutraceutical supports, to animals and human beings. Suitable unit administration forms comprise oral-route forms such as tablets, gel capsules, powders, granules and oral suspensions or solutions. The pharmaceutical or nutraceutical compositions may further contain antioxidant agents such as ascorbic acid, ascorbyl palmitate, BHT, potassium sorbate or *Rosmarinus officinalis* extracts. The pharmaceutical compositions may further contain flavour agents such as sugars, fruit or tea flavourings. Compositions comprising probiotics according to the invention can be prepared in water suitably mixed with a with a gelling agent, preferably modified starch. In one embodiment, the vehicle further comprises hydroxypropylmethylcellulose. In one embodiment, the vehicle does not comprise hydroxypropylmethylcellulose. Prolonged absorption of the injectable compositions can be brought about by the use in the compositions of agents delaying absorption, namely coatings as hereinafter described. The person responsible for administration will, in any event, determine the appropriate dose for the individual subject.
- **"zinc bisglycinate"** or **"zinc glycinate"** or "zinc bisglycinate chelate" designates zinc;2-aminoacetate C₄H₈N₂O₄Zn.CAS number 14281-83-5.

### DETAILED DESCRIPTION

The Applicant has demonstrated that particular zinc and chromium salts do not affect the *Hafnia alvei* viability. Consequently, the present invention paves the way for probiotic composition of *Hafnia alvei* further comprising zinc and or/chromium. The latter mineral elements being well established in the art as oligominerals necessary for the prevention, treatment or improvement of metabolic conditions.

This invention relates to a pharmaceutical or nutraceutical composition, comprising a *Hafnia alvei* strain probiotic composition; said strain expressing the ClpB protein; in association with zinc bisglycinate and/or chrome picolinate.

### Organic sources of Zn and Cr

According to a first embodiment, the pharmaceutical or nutraceutical composition, comprises only zinc bisglycinate
According to a second embodiment, the pharmaceutical or nutraceutical composition, comprises only chrome picolinate.
According to a preferred embodiment, the pharmaceutical or nutraceutical composition, comprises zinc bisglycinate and chrome picolinate.

In one embodiment, chrome picolinate is in an amount ranging from 0.01 to 0.04 % (w/w), in weight relative to the composition. In one embodiment, chrome picolinate is in an amount ranging from 0.01 to 0.03 % (w/w), in weight relative to the composition. In one embodiment, chrome picolinate is in an amount of about 0.01 %, about 0.02 % or about 0.03 % (w/w), in weight relative to the composition. In a preferred embodiment, chrome picolinate is in an amount of about 0.02 %, in weight relative to the composition.

In one embodiment, zinc bisglycinate is in an amount ranging from 2.0 to 4.0 % (w/w), in weight relative to the composition. In one embodiment, zinc bisglycinate is in an amount ranging from 2.0 to 3.0 % (w/w), in weight relative to the composition. In one embodiment, zinc bisglycinate is in an amount of about 2.5 %, about 2.8 % or about 3.0 % (w/w), in weight relative to the composition. In a preferred embodiment, zinc bisglycinate is in an amount of about 2.8 %, in weight relative to the composition.

In one embodiment, zinc bisglycinate is in an amount ranging from 2.0 to 3.0 % (w/w) and zinc bisglycinate is in an amount ranging from 2.0 to 3.0 % (w/w), in weight relative to the composition.

### Hafnia alvei strain probiotic composition

In one embodiment, the probiotic composition essentially consists of or comprises at least 75 % (w/w) *Hafnia alvei* probiotic strain. In one embodiment, the composition comprises at least 75 %, at least 80 % (w/w), at least 85 % (w/w), at least 90 % (w/w), at least 95 %, at least 96 %, at least 97 %, at least 98 % (w/w) or, at least 99 % of a probiotic strain, preferably *Hafnia alvei* probiotic strain.

In one embodiment, the probiotic composition is a solid composition.

In one preferred embodiment, the probiotic composition is a pulverulent composition (powder).

In one embodiment, the pulverulent composition (powder) presents a particle size distribution wherein particles smaller than 500 µm represent less than 80 % of the particle size distribution.

In one particular embodiment, the probiotic composition is a freeze-dried composition.

In one embodiment, the probiotic composition presents more than 95 % (w/w) of dry matter, in weight relative to the total composition.

In one preferred embodiment, the probiotic composition presents a water activity value (Aw) not exceeding 0.05, preferably not exceeding 0.03, even more preferably not exceeding 0.02.

### Hafnia alvei

*Hafnia alvei* is a facultatively anaerobic rod-shaped bacillus belonging to the family of Enterobacteriaceae.

In one embodiment, *Hafnia alvei* is a food-grade *Hafnia alvei* strain.

In one embodiment, *Hafnia alvei* is *Hafnia alvei* 4597 strain.

### ClpB

WO2017/174658 describes that *Hafnia alvei* is a ClpB-protein-expressing probiotic strain.

As used herein, the term "ClpB" has its general meaning in the art and is also known as heat shock protein F84.1 which is a member of the Hsp100/ClpB family of hexameric AAA+-ATPases. ClpB has been described as an essential factor for acquired thermotolerance several Gram-negative and Gram-positive bacteria. Typically, the amino acid sequence of chaperone protein ClpB comprises or consists of an amino acid sequence 96 to 100% identical to the amino acid sequence of SEQ ID NO: 1. Preferably, the amino acid sequence of ClpB is 96, 97, 98, 99 or 100% identical to the amino acid sequence 540-550 (ARWTGIPVSR) of SEQ ID NO: 1.

In one embodiment, the ClpB protein designates the 96 kDa peptide of SEQ ID NO: 1.

In the context of the present application, the percentage of identity is calculated using a global alignment (i.e. the two sequences are compared over their entire length). Methods for comparing the identity of two or more sequences are well known in the art. The «needle» program, which uses the Needleman-Wunsch global alignment algorithm (Needleman and Wunsch, 1970 J. Mol. Biol. 48:443-453) to find the optimum alignment (including gaps) of two sequences when considering their entire length, may for example be used. The needle program is, for example, available on the ebi.ac.uk world wide web site. The percentage of identity in accordance with the invention is preferably calculated using the EMBOSS: needle (global) program with a "Gap Open" parameter equal to 10.0, a "Gap Extend" parameter equal to 0.5, and a Blosum62 matrix.

According to the invention the ClpB protein mimic the alpha-MSH protein for inducing satiation. Thus, in some embodiments, the ClpB protein of the present invention is recognized by an anti-alpha-MSH antibody.

In one embodiment, the ClpB protein designates the 96 kDa peptide of SEQ ID NO: 1.

In one embodiment, the ClpB protein designates the ClpB fragments of 70, 60, 45, 40, 37, 35, 25 and 17 kDa fragments. Such fragments are recognized by an anti-alpha-MSH antibody. In one embodiment, the ClpB fragments are selected from the fragments of 70, 40, 37 and 25 kDa fragments.

In one embodiment, the ClpB protein designates alpha-MSH antibody cross-reacting dimers or precursors of ClpB and fragments thereof. In one embodiment, such dimers or precursors are selected from the fragments of 100, 125, 130 and 150 kDa.

Typically, the antibody is a monoclonal antibody. In some embodiments, the antibody is a polyclonal antibody such as polyclonal rabbit anti-α-MSH IgG (1:1000, Peninsula Laboratories, San Carlos, CA, USA). The amino acid sequence of α-MSH preferably comprises or consists of the amino acid sequence SYSMEHFRWGKPV (SEQ ID NO: 2) (Gen Pept Sequence ID, PRF: 223274, as available on December 2, 2013).

SEQ ID NO: 1:

MRLDRLTNKF QLALADAQSL ALGHDNQFIE PLHLMSALLN QEGGSVSPLL
TSAGINAGQL RTDINQALNR LPQVEGTGGD VQPSQDLVRV LNLCDKLAQK
RGDNFISSEL FVLAALESRG TLADILKAAG ATTANITQAI EQMRGGESVN
DQGAEDQRQA LKKYTIDLTE RAEQGKLDPV IGRDEEIRRT IQVLQRRTKN
NPVLIGEPGV GKTAIVEGLA QRIINGEVPE GLKGRRVLAL DMGALVAGAK
YRGEFEERLK GVLNDLAKQE GNVILFIDEL HTMVGAGKAD GAMDAGNMLK
PALARGELHC VGATTLDEYR QYIEKDAALE RRFQKVFVAE PSVEDTIAIL
RGLKERYELH HHVQITDPAI VAAATLSHRY IADRQLPDKA IDLIDEAASS
IRMQIDSKPE ELDRLDRRII QLKLEQQALM KESDEASKKR LDMLNEELSD
KERQYSELEE EWKAEKASLS GTQTIKAELE QAKIAIEQAR RVGDLARMSE
LQYGKIPELE KQLEAATQLE GKTMRLLRNK VTDAEIAEVL ARWTGIPVSR

MMESEREKLL RMEQELHHRV IGQNEAVDAV SNAIRRSRAG LADPNRPIGS
FLFLGPTGVG KTELCKALAN FMFDSDEAMV RIDMSEFMEK HSVSRLVGAP
PGYVGYEEGG YLTEAVRRRP YSVILLDEVE KAHPDVFNIL LQVLDDGRLT
DGQGRTVDFR NTVVIMTSNL GSDLIQERFG ELDYAHMKEL VLGVVSHNFR
PEFINRIDEV VVFHPLGEQH IASIAQIQLK RLYKRLEERG YEIHISDEAL
KLLSENGYDP VYGARPLKRA IQQQIENPLA QQILSGELVP GKVIRLEVNE
DRIVAVQ

As used herein, "amino acids" are represented by their full name, their three letter code or their one letter code as well known in the art. Amino acid residues in peptides are abbreviated as follows: Phenylalanine is Phe or F; Leucine is Leu or L; Isoleucine is Ile or I; Methionine is Met or M; Valine is Val or V; Serine is Ser or S; Proline is Pro or P; Threonine is Thr or T; Alanine is Ala or A; Tyrosine is Tyr or Y; Histidine is His or H; Glutamine is Gln or Q; Asparagine is Asn or N; Lysine is Lys or K; Aspartic Acid is Asp or D; Glutamic Acid is Glu or E; Cysteine is Cys or C; Tryptophan is Trp or W; Arginine is Arg or R; and Glycine is Gly or G.

As used herein, the term "amino acids" includes both natural and synthetic amino acids, and both D and L amino acids. "Standard amino acid" or "naturally occurring amino acid" means any of the twenty standard L-amino acids commonly found in naturally occurring peptides. "Nonstandard amino acid residue" means any amino acid, other than the standard amino acids, regardless of whether it is prepared synthetically or derived from a natural source. For example, naphtlylalanine can be substituted for tryptophan to facilitate synthesis. Other synthetic amino acids that can be substituted include, but are not limited to, L-hydroxypropyl, L-3,4-dihydroxyphenylalanyl, alpha-amino acids such as L-alpha-hydroxylysyl and D-alpha-methylalanyl, L-alpha-methylalanyl, beta-amino acids, and isoquinolyl.

As used herein, "amino acid" also encompasses chemically modified amino acids, including but not limited to salts, amino acid derivatives (such as amides), and substitutions. Amino acids contained within the polypeptides of the present invention, and particularly at the carboxy- or amino-terminus, can be modified by methylation, amidation, acetylation or substitution with other chemical groups which can change the polypeptide's circulating half-life without adversely affecting their activity. Additionally, a disulfide linkage may be present or absent in the polypeptides of the invention.

### ClpB amount

Preferably the probiotic composition of *Hafnia alvei* that comprises ClpB protein in an amount of at least 0.7 % (w/w) in weight relative to the total weight of the probiotic composition. Typically, the ClpB protein is in an amount equal or superior to 0.7 % (w/w), preferably equal or superior to 0.8 % (w/w), even more preferably equal or superior to 0.9 % (w/w) in weight relative to the total weight of the probiotic composition.

In one embodiment, the ClpB protein is in an amount ranging:
- from 0.7 % to 2.0 (w/w),
- from 0.8 % to 2.0 (w/w),
- from 0.8 % to 1.8 (w/w),
- from 0.8 % to 1.5 (w/w),
- from 0.9 % to 2.0 (w/w),
- from 0.9 % to 1.8 (w/w), or
- from 0.9 % to 1.5 (w/w),
in weight relative to the total weight of the probiotic composition.

Preliminary data have shown that the biological effects of *Hafnia alvei* are CFU (Colony Forming Units)-dependent and total number *Hafnia alvei* cell number-dependent.

Thus, the probiotic composition may be further characterized by the number of *Hafnia alvei* Colony Forming Units as well as the total number *Hafnia alvei* cell number.

### Ratio

In one preferred embodiment, the ratio of the total number of *Hafnia alvei* Colony Forming Units to the total *Hafnia alvei* cell number is at least 10⁻⁴. In one embodiment, the ratio is at least 2.2 10⁻⁴, preferably at least 2.5 10⁻⁴, at least 3 10⁻⁴ or at least 5 10⁻³.

In one embodiment, the CFU to the total Hafnia alvei cell number ranges from 10⁻⁴ to 1.

In one embodiment, the CFU to the total Hafnia alvei cell number ranges from 5 10⁻⁴ to 1.

In one embodiment, the CFU to the total Hafnia alvei cell number ranges from 10⁻⁴ to 0.5. In one embodiment, the CFU to the total Hafnia alvei cell number ranges from 5 10⁻⁴ to 0.5.

In one preferred embodiment, the CFU to the total Hafnia alvei cell number ranges from 10⁻⁴ to 0.8.

Without willing to be bound by a theory, the ratio according to the present invention guarantees the optimal ClpB secretion by *Hafnia alvei* within the intestinal tract of the subject that consumed the composition according to the invention. Thus, *Hafnia alvei* strains may have a dual role. Firstly, acting as a protective vehicle for the ClpB that was expressed by the strain prior to its administration to the subject. Secondly, the *Hafnia alvei* forming part of the subject's microbiota, shall continue secreting ClpB under the suitable conditions (stationary phase of the strain's growth phase). It appears that the *Hafnia alvei* Colony Forming Units to the total *Hafnia alvei* cell number optimizes said dual role of *Hafnia alvei* and concomitantly the desired beneficial effects on body weight control.

### CFU

In one embodiment, the number of *Hafnia alvei* Colony Forming Units cells is equal or superior to 10⁶ per gram of the probiotic composition. In one embodiment, the number of *Hafnia alvei* Colony Forming Units cells is equal or superior to 5 10⁶ per gram of the probiotic composition. In one embodiment, the number of *Hafnia alvei* Colony Forming Units cells is equal or superior to 10⁷ per gram of the probiotic composition. In one embodiment, the number of *Hafnia alvei* Colony Forming Units cells is equal or superior to 5 10⁷ per gram of the probiotic composition. In one embodiment, the number of *Hafnia alvei* Colony Forming Units cells is equal or superior to 10⁸ per gram of the probiotic composition. In one embodiment, the number of *Hafnia alvei* Colony Forming Units cells is equal or superior to 5 10⁸ per gram of the probiotic composition. In one embodiment, the number of *Hafnia alvei* Colony Forming Units cells is equal or superior to 10⁹ per gram of the probiotic composition. In one embodiment, the number of *Hafnia alvei* Colony Forming Units cells is equal or superior to 10¹⁰ per gram of the probiotic composition. In one embodiment, the number of *Hafnia alvei* Colony Forming Units cells is equal or superior to 10¹¹ per gram of the probiotic composition

In one embodiment, the number of *Hafnia alvei* Colony Forming Units cells ranges from about 10⁶ to about 5 10¹¹ about per gram of the probiotic composition.

In one embodiment, the number of *Hafnia alvei* Colony Forming Units cells ranges from about 10⁷ to about 5 10¹¹ about per gram of the probiotic composition.

In one embodiment, the number of *Hafnia alvei* Colony Forming Units cells ranges from about 10⁷ to about 10¹¹ about per gram of the probiotic composition.

In one embodiment, the number of *Hafnia alvei* Colony Forming Units cells ranges from about 10⁶ to about 10⁹ about per gram of the probiotic composition.

In one embodiment, the number of *Hafnia alvei* Colony Forming Units cells ranges from about 10⁷ to about 5 10¹¹ about per gram of the probiotic composition.

In one embodiment, the number of *Hafnia alvei* Colony Forming Units cells ranges from about 10⁷ to about 10¹¹ about per gram of the probiotic composition.

CFU count techniques are generally known in the art. In one embodiment, the number of CFU is calculated by counting colonies on petri dishes.

### Total cell number

One skilled in the art can calculate the total number *Hafnia alvei* cell number based on the CFU number and the ratio of CFU to the total number *Hafnia alvei* cell number, as previously described.

In one embodiment, the total number *Hafnia alvei* cell number is at least 10⁸ per gram of the probiotic composition.

In one embodiment, the total number *Hafnia alvei* cell number is at least 10⁹ per gram of the probiotic composition.

In one preferred embodiment, the total number *Hafnia alvei* cell number is at least 10¹⁰ per gram of the probiotic composition.

In one embodiment, the total number *Hafnia alvei* cell number is at least 5 10¹⁰ per gram of the probiotic composition.

In one embodiment, the total number *Hafnia alvei* cell number is equal or superior to 10¹¹ per gram of the probiotic composition.

In one embodiment, the total number *Hafnia alvei* cell number ranges from 10⁸ to 10¹¹ per gram of the probiotic composition.

In one embodiment, the total number *Hafnia alvei* cell number ranges from 10⁹ to 10¹¹ per gram of the probiotic composition.

In one embodiment, the total number *Hafnia alvei* cell number ranges from 10¹⁰ to 10¹¹ per gram of the probiotic composition.

In one embodiment, the total number *Hafnia alvei* cell number is about 10⁸, about 10⁹, about 10¹⁰, about 10¹¹ or about 10¹², per gram of the probiotic composition.

In one embodiment, the total number *Hafnia alvei* cells comprises alive *Hafnia alvei* cells, alive but inactive *Hafnia alvei* cells, disrupted *Hafnia alvei* cells, dead *Hafnia alvei* cells and mixtures thereof.

In one embodiment, the total number *Hafnia alvei* cells is measured by Flow Cytometry. According to such embodiment the total number *Hafnia alvei* cells comprise, intact *Hafnia alvei* cells, disrupted *Hafnia alvei* cells, dead *Hafnia alvei* cells and mixtures thereof.

In one embodiment, the total number *Hafnia alvei* cells comprises at least 45 % of intact *Hafnia alvei* cells relative to the total cell population. In one embodiment, the total number *Hafnia alvei* cells comprises at least 50 % of intact *Hafnia alvei* cells relative to the total cell population. In one embodiment, the total number *Hafnia alvei* cells comprises at least 65 % of intact *Hafnia alvei* cells relative to the total cell population.

In one embodiment, the total number *Hafnia alvei* cells comprises at least 45 % of intact *Hafnia alvei* cells and less than 5% of dead *Hafnia alvei* cells, relative to the total cell population.

In one embodiment, the total number *Hafnia alvei* cells comprises at least 50 % of intact *Hafnia alvei* cells and less than 5% of dead *Hafnia alvei* cells, relative to the total cell population.

In one embodiment, the total number *Hafnia alvei* cells comprises at least 65 % of intact *Hafnia alvei* cells and less than 5% of dead *Hafnia alvei* cells, relative to the total cell population.

In one embodiment, the total number *Hafnia alvei* cells comprises at least 45 % of intact *Hafnia alvei* cells and less than 3% of dead *Hafnia alvei* cells, relative to the total cell population.

In one embodiment, the total number *Hafnia alvei* cells comprises at least 50 % of intact *Hafnia alvei* cells and less than 3% of dead *Hafnia alvei* cells, relative to the total cell population.

In one embodiment, the total number *Hafnia alvei* cells comprises at least 65 % of intact *Hafnia alvei* cells and less than 3% of dead *Hafnia alvei* cells, relative to the total cell population.

### Food composition

In one embodiment, pharmaceutical or nutraceutical composition comprises at least 5 % (w/w) of the previously described probiotic composition, in weight relative to the total pharmaceutical or nutraceutical composition.

In one embodiment, pharmaceutical or nutraceutical composition comprises at least 8 % (w/w) of the previously described probiotic composition, in weight relative to the total pharmaceutical or nutraceutical composition.

In one embodiment, pharmaceutical or nutraceutical composition comprises at least 10 % (w/w) of the previously described probiotic composition, in weight relative to the total pharmaceutical or nutraceutical composition.

In one embodiment, pharmaceutical or nutraceutical composition comprises from 5 % to 30 %(w/w) of the previously described probiotic composition, in weight relative to the total pharmaceutical or nutraceutical composition.

In one embodiment, pharmaceutical or nutraceutical composition comprises from 8 % to 20 %(w/w) of the previously described probiotic composition, in weight relative to the total pharmaceutical or nutraceutical composition.

In one embodiment, pharmaceutical or nutraceutical composition comprises from 10 % to 15 %(w/w) of the previously described probiotic composition, in weight relative to the total pharmaceutical or nutraceutical composition.

In one embodiment, pharmaceutical or nutraceutical composition comprises about 9 %, about 10 %, about 11 %, about 12 %, about 13 %, about 14 %, or about 15 %(w/w) of the previously described probiotic composition, in weight relative to the total pharmaceutical or nutraceutical composition. In one embodiment, pharmaceutical or nutraceutical composition comprises about 10 %, about 11 % or about 12 %, (w/w) of the previously described probiotic composition, in weight relative to the total pharmaceutical or nutraceutical composition.

### Excipient

In one embodiment, the pharmaceutical or nutraceutical composition of the invention further comprises at least one pharmaceutically or nutraceutically acceptable excipient.

The pharmaceutical or nutraceutical composition that comprises the bacterial strain, in particular the probiotic bacterial strain, of the present invention typically comprises carriers or vehicles. "Carriers" or "vehicles" mean materials suitable for administration and include any such material known in the art such as, for example, any liquid, gel, solvent, liquid diluent, solubilizer, or the like, which is non-toxic and which does not interact with any components, in particular with the bacterial strain, of the composition in a deleterious manner. Examples of pharmaceutically or nutraceutically acceptable carriers include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, hydroxypropylmethyl-cellulose polyvinylpyrrolidone, and the like.

Preliminary results showed that *Hafnia alvei* strain viability is reduced in the acidic conditions of the stomach.

Thus, the pharmaceutical or nutraceutical composition may further comprise a texturizing agent, preferably a gelling agent, even more preferably a modified starch to protect the probiotic strain from the gastric acid degradation.

In one embodiment, the at least one pharmaceutically or nutraceutically acceptable excipient is a vehicle selected from modified starches. In one embodiment, the vehicle is a pre-gelatinized starch. In one embodiment, the vehicle is a modified maize starch. In one embodiment, the vehicle is a pre-gelatinized maize starch, such as for example Pregeflo ^{®}.

In one embodiment, the at least one pharmaceutically or nutraceutically acceptable excipient is not a gelling agent comprising hydroxypropylmethylcellulose.

In one embodiment, the vehicle is in an amount ranging from 70 % to 90 % (w/w), in weight relative to the total pharmaceutical or nutraceutical composition.

In one embodiment, the vehicle is pre-gelatinized starch in an amount ranging from 70 % to 88 % (w/w), in weight relative to the total pharmaceutical or nutraceutical composition.

In one embodiment, the vehicle is pre-gelatinized starch in an amount ranging from 80 % to 88 % (w/w), in weight relative to the total pharmaceutical or nutraceutical composition.

In one embodiment, the vehicle is pre-gelatinized starch in an amount of about 81 %, about 82 %, about 83 %, about 84 %, about 85 %, about 86 %, about 87 %, or about 88 % (w/w), in weight relative to the total pharmaceutical or nutraceutical composition.

The pharmaceutical or nutraceutical composition may further comprise an anti-adherent agent in order to improve the rheological properties of the pharmaceutical or nutraceutical composition.

In one embodiment, the pharmaceutical or nutraceutical composition comprises at least 0.5 % (w/w) of an anti-adherent agent, in weight relative to the total pharmaceutical or nutraceutical composition.

In one embodiment, the anti-adherent agent is magnesium stearate.

In one embodiment, the pharmaceutical or nutraceutical composition comprises about 0.5 %, about 0.7 %, about 0.8 %, about 1.0 %, about 1.2 % or about 1.5 %, (w/w) of an anti-adherent agent, preferably magnesium stearate. In one embodiment, the pharmaceutical or nutraceutical composition comprises about 1.0 % (w/w) of magnesium stearate, in weight relative to the total pharmaceutical or nutraceutical composition.

In one embodiment, the pharmaceutical or nutraceutical composition further comprises minerals and micronutrients such as trace elements and vitamins in accordance with the recommendations of Government bodies such as the USRDA. For example, the composition may contain per daily dose one or more of the following micronutrients zinc, chrome, calcium, magnesium, phosphorus, iron, copper, iodine selenium, beta carotene, Vitamin C, Vitamin B1, Vitamin B6 Vitamin B2, niacin, Vitamin B12, folic acid, biotin, Vitamin D or Vitamin E.

In one embodiment, the pharmaceutical or nutraceutical composition further comprises at least one prebiotic. "Prebiotic" means food substances intended to promote the growth of the probiotic bacterial strain of the present invention in the intestines. The prebiotic may be selected from the group consisting of oligosaccharides and optionally contains fructose, galactose, mannose, soy and/or inulin; and/or dietary fibers.

In one embodiment, the pharmaceutical or nutraceutical composition comprises:
- from 10% to 15 %(w/w) of a *Hafnia alvei* probiotic strain composition as previously described;
- from 80 to 85 % (w/w) of modified starch;
- from 0.5 to 1.5 % (w/w) of magnesium stearate;
- from 2.0 to 3.0 % (w/w) of zinc bisglycinate; and
- from 0.01 to 0.03 % (w/w) chrome picolinate:
in weight relative to the total weight of the composition.

With the proviso that the total in weight percentage concentrations do not exceed 100%. One skilled in the art can adapt the concentration of each ingredient with in the disclosed ranges so as not to exceed 100%.

In one embodiment, the pharmaceutical or nutraceutical composition comprises:
- about 11% (w/w) of a *Hafnia alvei* probiotic strain composition of the invention;
- about 85 % (w/w) of modified starch;
- about 1% (w/w) of magnesium stearate;
- about 2.8 % (w/w) of zinc bisglycinate; and
- about 0.02 % (w/w) chrome picolinate;
in weight relative to the total weight of the composition.

### Oral dosage form

In a further aspect, the invention relates to oral dosage forms comprising the pharmaceutical or nutraceutical composition as previously described.

In one embodiment, the oral dosage form is selected from tablets and capsules.

In one embodiment, the oral dosage form is coated with an enteric coating.

In one embodiment, the oral dosage form is selected from enterically-coated tablets and enterically-coated capsules.

Suitable coatings for such dosage forms are generally known in the art. In one embodiment, the enteric-coating is selected from Methyl acrylate-methacrylic acid copolymers, Cellulose acetate phthalate (CAP), Cellulose acetate succinate, Hydroxypropyl methyl cellulose phthalate, Hydroxypropyl methyl cellulose acetate succinate (hypromellose acetate succinate), Polyvinyl acetate phthalate (PVAP), Methyl methacrylate-methacrylic acid copolymers, shellac, cellulose acetate trimellitate, Sodium alginate and zein. In one embodiment, the enteric-coating may further comprise a thickening agent selected from starches, pectins and polysaccharides selected from algicinic acid and salts thereof, agar-agar, gelatin, carrageenan, locust vena gum and gellan gum.

The Applicants found out that the enteric coating comprising Hydroxypropyl methyl cellulose and gellan gum is particularly advantageous. Indeed, enteric-coated capsules according to the invention provided an improved stability to the bioactive ClpB and fragments thereof, compared to standard Hydroxypropyl methyl cellulose enteric-coatings.

In one preferred embodiment, the oral dosage form is selected from enterically-coated tablets and enterically-coated capsules, wherein the enteric-coating is a mixture comprising Hydroxypropyl methyl cellulose and gellan gum.

In one preferred embodiment, the oral dosage form is an enterically-coated capsule, wherein the enteric-coating is a mixture comprising Hydroxypropyl methyl cellulose and gellan gum.

In one preferred embodiment, the oral dosage form is an enterically-coated capsule, wherein the enteric-coating is a mixture comprising Hydroxypropyl methyl cellulose and gellan gum.

In one embodiment, the enteric coating is the capsule itself.

In one embodiment, the enteric coating comprises from 85 to 95 % Hydroxypropyl methyl cellulose and from 5 to 15% gellan gum (w/w) in weight relative to the enteric-coating or the capsule weight.

In one embodiment, the enteric coating comprises about 95 % Hydroxypropyl methyl cellulose and about 5% gellan gum (w/w) in weight relative to the enteric-coating or the capsule weight.

In one embodiment, the enteric-coating is a DRcaps ^{™} capsule commercialized by Capsugel^{®}.

In a last aspect, the invention relates to a blister comprising at least one oral dosage form as previously described.

In one embodiment, the blister comprises at least one capsule as previously described. In one embodiment, the blister comprises 5, 10, 15, 20, 25, 30, 35, 40, 45, 50, 55, or 60 capsules as previously described.

In one embodiment, the blister comprises 30 capsules as previously described.

One further aspect of the present invention, which is not claimed, relates to a method of:
- reducing fat mass on lean mass ratio
- reducing food intake,
- inducing satiation,
- stimulating weight loss, or
- limiting weight gain
in a subject in need thereof comprising administering to the subject an effective amount of the composition, the pharmaceutical or nutraceutical composition or the oral dosage form according to the invention.

In one embodiment, the method is a non-therapeutic method.

### EXAMPLES

The present invention is further illustrated by the following examples.

### Example 1: Liquid media preparation

Different sources of zinc and chrome where evaluated for their impact on the *Hafnia alvei* probiotic strain cell integrity. Firstly, the cell integrity of *Hafnia alvei* was evaluated in liquid media.

The concentration of the minerals were based on their estimated concentration in ~120 mL (typical glass of water). However, in view of observing the *Hafnia alvei* strain development, the the zinc and chrome sources were solubilized in a Luria Browth medium (LB). The composition of LB is as follows: Tryptone 10g/L, yeast extract 5 g/L (Biokar Diagnostic^{®}) and NaCl 0.5g/L (Fluka^{®}).

The following test media were prepared as presented in table 1.

**Table 1. composition of test Hafnia alvei culture media.**

| Test medium | Mineral source | Mineral source concentration (µg/mL) |
|---|---|---|
| 1 | LB medium, control | - |
| 2 | LB medium, Chromium picolinate | 0.7 |
| 3 | LB medium, Chromium Hypori^{®} | 9.3 |
| 4 | LB medium, Zinc bisglycinate | 104 |
| 5 | LB medium, Zinc Hypori^{®} | 189 |
| 6 | LB medium, Zinc Gluconate | 148 |

Hypori^{®} zinc and chromium compositions comprise hydrolyzed rice proteins as carriers for zinc and chromium respectively. Hypori^{®} are commercialized by Pileje Industrie^{®}.

### Example 2: Evaluation of turbidity

*Hafnia alvei* 4597 was incubated with Test medial-6 of example 1 and the turbidity of the culture media was monitored during 48 hours by means of optical densitometry (OD at 640nm).

### 1. Chromium sources:

Chromium picolinate solution induced the same turbidity as the control (LB) medium.

Consequently, Chromium picolinate does not influence the strain development.

On the contrary, inferior OD values were observed with Chromium Hypori^{®} mineral source, implying that Chromium Hypori^{®} inhibits the development of *Hafnia alvei* strain.

### 2. Zinc sources:

No significant differences were observed among the turbidity of the cultures of media 1 (control, LB) and 4-6 (zinc comprising media). Further investigation was carried out by means of flow cytometry.

### Example 3: Flow cytometry

Samples of the culture media 1-6 were further assessed for the cell integrity by means of flow cytometry using as markers propidium iodide and Syto^{®} 24. The flow cytometry measured the number of intact cells opposed to the population of cells whose cellular membrane integrity had been compromised.

### 1. Chromium sources:

The flow cytometry assay confirmed the observations of Example 2. Indeed, chromium picolinate comprising medium induced the same concentration of intact cells per g of culture medium as the control solution (about 1.1 10⁹ at 24 hours of incubation).

Consequently, Chromium picolinate does not influence the strain development.

On the contrary, Chromium Hypori^{®} mineral source induced a considerable reduction of intact cells per g of culture medium compared to the control medium (about 9 10⁸ at 24h for medium 5).

Chromium picolinate was retained as the optimal candidate for the compositions of the invention.

### 2. Zinc sources:

The results relative to the effect of zinc comprising media are presented in table 2.

**Table 2. Flow cytometry results at 24h for the zinc comprising media.**

| Test solution | Zinc source | Intact cells/g of medium |
|---|---|---|
| 1 | LB medium, control | 1.1 10⁹ |
| 4 | Zinc bisglycinate | 1.0 10⁹ |
| 5 | Zinc Hypori^{®} | 9.0 10⁸ |
| 6 | Zinc Gluconate | 7.5 10⁸ |

Zinc bisglycinate was proven to induce no significant development inhibition compared to the control medium, as opposed to gluconate and Hypori^{®} zinc sources.

### Example 4: Zinc and chrome association with Hafnia alvei

Given the results of examples 2 and 3, a new test medium was prepared. The new medium (medium 7) comprises LB medium as previously described, 0.7 µg/mL of chromium picolinate and 104 µg/mL of zinc bisglycinate.

The development of *Hafnia alvei* in this new medium was monitored by optical densitometry and flow cytometry. LB medium with no added zinc or chrome was used as a control.

No reduction of the turbidity was observed with medium 7 compared to the control.

The absence of *Hafnia alvei* development inhibition was further confirmed by a flow cytometry analysis (about 1.2 10⁹ intact cells per g of culture medium at 24 hours of incubation for both control medium and medium 7).

### Example 5: Zinc and chrome association with Hafnia alvei in a solid composition

The following example shows that incubation of chromium picolinate and zinc bisglycinate with a *Hafnia alvei* powder does not inhibit the ulterior development of *Hafnia alvei.*

The probiotic composition according to table 3 was kept for 24 hours prior to the *Hafnia alvei* development assessment my measuring the number of the colony forming units (CFU). Results were compared with a *Hafnia alvei* probiotic composition that did not comprise any zinc or chromium sources (clinical batch).

| Ingredient | %(w/w) |
|---|---|
| *Hafnia alvei* 4597 | 11.2 |
| Pregeflo^{®} | 84.9 |
| Magnesium stearate | 1.0 |
| Chromium picolinate | 0.018 |
| Zinc bisglycinate | 2.8 |

After 24 h the CFU counting showed 2.7 10¹⁰ CFU per gram of the clinical batch probiotic composition.

Interestingly, after 24 h the CFU counting showed 4.2 10¹⁰ CFU per gram of the tested probiotic composition.

In conclusion, contrary to other chromium and zinc sources, chromium picolinate and zinc bisglycinate do not affect the growth of *Hafnia alvei* probiotic strain.

## Claims

1. A pharmaceutical or nutraceutical composition, comprising a *Hafnia alvei* strain probiotic composition; said strain expressing the ClpB protein; in association with zinc bisglycinate and/or chrome picolinate.

2. The pharmaceutical or nutraceutical composition according to claim 1, wherein chrome picolinate is in an amount ranging from 0.01 to 0.04 % (w/w), in weight relative to the composition.

3. The pharmaceutical or nutraceutical composition according to claim 1 or claim 2, wherein zinc bisglycinate is in an amount ranging from 2 to 4 % (w/w), in weight relative to the composition.

4. The pharmaceutical or nutraceutical composition according to any one of claims **1** to **3**, wherein:
- the ClpB protein in the *Hafnia alvei* probiotic composition is in an amount of at least 0.7 % (w/w) in weight relative to the total weight of said composition; and
- the ratio of the total number of *Hafnia alvei* Colony Forming Units to the total *Hafnia alvei* cell number is at least 10⁻⁴.

5. The pharmaceutical or nutraceutical composition according to claim **4**, wherein the number of *Hafnia alvei* Colony Forming Units cells is equal or superior to 10⁶ per gram of the probiotic composition.

6. The pharmaceutical or nutraceutical composition according to claim **4** or claim **5**, wherein the total number *Hafnia alvei* cell number is equal or superior to 10¹⁰ per gram of the probiotic composition.

7. The pharmaceutical or nutraceutical composition according to any one of claims **1** to **6**, wherein *Hafnia alvei* probiotic composition is freeze-dried.

8. The pharmaceutical or nutraceutical composition according to any one of claims **1** to **7**, said pharmaceutical or nutraceutical composition further comprising at least one pharmaceutically or nutraceutically acceptable excipient; preferably said excipient being selected from at least one anti-adherent and at least one texturizing agent.

9. The pharmaceutical or nutraceutical composition according to claim **8**, wherein said at least one anti-adherent is magnesium stearate.

10. The pharmaceutical or nutraceutical composition according to claim **8**, wherein said at least one texturizing agent is a modified starch.

11. The pharmaceutical or nutraceutical composition according to any one of claims **1** to **10**; said composition comprising:
- from 10% to 15 %(w/w) of a *Hafnia alvei* composition according to any one of claims **4** to **7**;
- from 80 to 85 % (w/w) of modified starch;
- from 0.5 to 1.5 % (w/w) of magnesium stearate;
- from 2.0 to 3.0 % (w/w) of a zinc organic salt selected from zinc bisglycinate; and
- from 0.01 to 0.03 % (w/w) of a chrome organic salt selected from chrome picolinate;
in weight relative to the total weight of the pharmaceutical or nutraceutical composition.

12. An oral dosage form selected from capsules and tablets, preferably capsules; said dosage form comprising the pharmaceutical or nutraceutical composition according to any one of claims **1** to **11**.

13. The oral dosage form according to claim **12**, said oral dosage form being coated with an enteric coating.

14. The oral dosage form according to claim **12** or claim **13**, said enteric coating comprising hydroxypropyl methyl-cellulose and gellan gum.

15. A blister comprising at least one oral dosage form according to any one of claims **12** to **14**.

## Patentansprüche

1. Pharmazeutische oder nutrazeutische Zusammensetzung, die eine probiotische Zusammensetzung mit einem *Hafnia alvei-Stamm;* wobei der Stamm das ClpB-Protein exprimiert; in Verbindung mit Zinkbisglycinat und/oder Chrompicolinat umfasst.

2. Pharmazeutische oder nutrazeutische Zusammensetzung nach Anspruch **1**, wobei Chrompicolinat, auf die Zusammensetzung bezogen, in einer Menge im Bereich von 0,01 bis 0,04 Gewichts-% (w/w) vorliegt.

3. Pharmazeutische oder nutrazeutische Zusammensetzung nach Anspruch **1** oder Anspruch 2, wobei Zinkbisglycinat, auf die Zusammensetzung bezogen, in einer Menge im Bereich von 2 bis 4 Gewichts-% (w/w) vorliegt.

4. Pharmazeutische oder nutrazeutische Zusammensetzung nach einem der Ansprüche **1** bis **3**, wobei:
- das ClpB-Protein in der probiotischen *Hafnia* alvei-Zusammensetzung, auf das Gesamtgewicht der Zusammensetzung bezogen, in einer Menge von mindestens 0,7 Gewichts-% (w/w) vorliegt; und
- das Verhältnis der Gesamtanzahl an *Hafnia* alvei-koloniebildenden Einheiten zur *Hafnia* alvei-Gesamtzellzahl mindestens 10⁻⁴ beträgt.

5. Pharmazeutische oder nutrazeutische Zusammensetzung nach Anspruch **4**, wobei die Anzahl an *Hafnia* alvei-koloniebildende Einheiten-Zellen pro Gramm der probiotischen Zusammensetzung gleich oder größer als 10⁶ ist.

6. Pharmazeutische oder nutrazeutische Zusammensetzung nach Anspruch **4** oder Anspruch **5**, wobei die Gesamtanzahl *Hafnia alvei-*Zellzahl pro Gramm der probiotischen Zusammensetzung gleich oder größer als 10¹⁰ ist.

7. Pharmazeutische oder nutrazeutische Zusammensetzung nach einem der Ansprüche **1** bis **6**, wobei die probiotische *Hafnia alvei*-Zusammensetzung gefriergetrocknet ist.

8. Pharmazeutische oder nutrazeutische Zusammensetzung nach einem der Ansprüche **1** bis **7**, wobei die pharmazeutische oder nutrazeutische Zusammensetzung weiter mindestens einen pharmazeutisch oder nutrazeutisch akzeptablen Hilfsstoff umfasst; wobei der Hilfsstoff vorzugsweise aus mindestens einem Antihaftmittel und mindestens einem Texturierungsmittel ausgewählt ist.

9. Pharmazeutische oder nutrazeutische Zusammensetzung nach Anspruch **8**, wobei das mindestens eine Antihaftmittel Magnesiumstearat ist.

10. Pharmazeutische oder nutrazeutische Zusammensetzung nach Anspruch **8**, wobei das mindestens eine Texturierungsmittel eine modifizierte Stärke ist.

11. Pharmazeutische oder nutrazeutische Zusammensetzung nach einem der Ansprüche **1** bis **10**; wobei die Zusammensetzung, auf das Gesamtgewicht der pharmazeutischen oder nutrazeutischen Zusammensetzung bezogen, umfasst:
- 10 Gewichts-% bis 15 Gewichts-% (w/w) einer *Hafnia alvei*-Zusammensetzung nach einem der Ansprüche **4** bis **7**;
- 80 bis 85 Gewichts-% (w/w) modifizierte Stärke;
- 0,5 bis 1,5 Gewichts-% (w/w) Magnesiumstearat;
- 2,0 bis 3,0 Gewichts-% (w/w) eines organischen Zinksalzes, ausgewählt aus Zinkbisglycinat; und
- 0,01 bis 0,03 Gewichts-% (w/w) eines organischen Chromsalzes, ausgewählt aus Chrompicolinat.

12. Orale Darreichungsform, ausgewählt aus Kapseln und Tabletten, vorzugsweise Kapseln; wobei die Darreichungsform die pharmazeutische oder nutrazeutische Zusammensetzung nach einem der Ansprüche **1** bis **11** umfasst.

13. Orale Darreichungsform nach Anspruch **12**, wobei die orale Darreichungsform mit einem magensaftresistenten Überzug überzogen ist.

14. Orale Darreichungsform nach Anspruch **12** oder Anspruch **13**, wobei der magensaftresistente Überzug Hydroxypropylmethylcellulose und Gellangummi umfasst.

15. Blister, der mindestens eine orale Darreichungsform nach einem der Ansprüche **12** bis **14** umfasst.

## Revendications

1. Composition pharmaceutique ou nutraceutique, comprenant une composition probiotique de souche *Hafnia alvei* ; ladite souche exprimant la protéine ClpB ; en association avec du bisglycinate de zinc et/ou du picolinate de chrome.

2. Composition pharmaceutique ou nutraceutique selon la revendication **1**, dans laquelle le picolinate de chrome est présent en une quantité allant de 0,01 à 0,04 % (p/p), en poids par rapport à la composition.

3. Composition pharmaceutique ou nutraceutique selon la revendication **1** ou la revendication **2**, dans laquelle le bisglycinate de zinc est présent en une quantité allant de 2 à 4 % (p/p), en poids par rapport à la composition.

4. Composition pharmaceutique ou nutraceutique selon l'une quelconque des revendications **1** à **3**, dans laquelle :
- la protéine ClpB dans la composition probiotique de souche *Hafnia alvei* est en une quantité d'au moins 0,7 % (p/p) en poids par rapport au poids total de ladite composition ; et
- le rapport du nombre total d'Unités Formant Colonie de *Hafnia alvei* au nombre total de cellules de *Hafnia alvei* est d'au moins 10⁻⁴.

5. Composition pharmaceutique ou nutraceutique selon la revendication **4**, dans laquelle le nombre de cellules d'Unités Formant Colonie de *Hafnia alvei* est égal ou supérieur à 10⁶ par gramme de la composition probiotique.

6. Composition pharmaceutique ou nutraceutique selon la revendication **4** ou la revendication **5**, dans laquelle le nombre total de cellules de *Hafnia alvei* est égal ou supérieur à 10¹⁰ par gramme de la composition probiotique.

7. Composition pharmaceutique ou nutraceutique selon l'une quelconque des revendications **1** à **6**, dans laquelle la composition probiotique de *Hafnia alvei* est lyophilisée.

8. Composition pharmaceutique ou nutraceutique selon l'une quelconque des revendications **1** à **7**, ladite composition pharmaceutique ou nutraceutique comprenant en outre au moins un excipient pharmaceutiquement ou nutraceutiquement acceptable ; de préférence ledit excipient étant choisi parmi au moins un anti-adhérent et au moins un agent texturant.

9. Composition pharmaceutique ou nutraceutique selon la revendication **8**, dans laquelle ledit au moins un anti-adhérent est le stéarate de magnésium.

10. Composition pharmaceutique ou nutraceutique selon la revendication **8**, dans laquelle ledit au moins un agent texturant est un amidon modifié.

11. Composition pharmaceutique ou nutraceutique selon l'une quelconque des revendications **1** à **10** ; ladite composition comprenant :
- de 10 % à 15 % (p/p) d'une composition de *Hafnia alvei* selon l'une quelconque des revendications **4** à **7** ;
- de 80 à 85 % (p/p) d'amidon modifié ;
- de 0,5 à 1,5 % (p/p) de stéarate de magnésium ;
- de 2,0 à 3,0 % (p/p) d'un sel organique d'acide choisi parmi le bisglycinate de zinc ; et
- de 0,01 à 0,03 % (p/p) d'un sel organique de chrome choisi parmi le picolinate de chrome ;
en poids par rapport au poids total de la composition pharmaceutique ou nutraceutique.

12. Une forme galénique orale choisie parmi les gélules et les comprimés, de préférence les gélules ; ladite forme galénique comprenant la composition pharmaceutique ou nutraceutique selon l'une quelconque des revendications **1** à **11**.

13. Forme galénique orale selon la revendication **12,** ladite forme galénique orale étant enrobée d'un enrobage entérique.

14. Forme galénique orale selon la revendication **12** ou la revendication **13**, ledit enrobage entérique comprenant de l'hydroxypropylméthylcellulose et de la gomme gellane.

15. Plaquette thermoformée comprenant au moins une forme galénique orale selon l'une quelconque des revendications **12 à 14**.
